# EUROPEAN PATENT SPECIFICATION

(11) **EP 0 342 203 B1**
(45) Date of publication and mention of the grant of the patent: **06.07.1994**
(21) Application number: 88902532.6
(22) Date of filing: 23.03.1988
(51) Int. Cl.: C07D 405/04, C07D 473/34

(54) **2',3' DIDEOXYRIBOFURANOXIDE DERIVATIVES**
2',3'-DIDEOXYRIBOFURANOXID-DERIVATE
DERIVES D'OXYDE DE 2',3' DIDESOXYRIBOFURANNE

(30) Priority: 24.03.1987 GB 8706991; 09.06.1987 GB 8713464
(43) Date of publication of application: 23.11.1989
(73) Proprietor: NYCOMED IMAGING AS, 0401 Oslo 4 (NO)
(72) Inventor: KLAVENESS, Jo, N-0276 Oslo 2 (NO); RISE, Frode, S-752 63 Uppsala (SE); UNDHEIM, Kjell, N-1310 Blommenholm (NO)
(74) Representative: Matthews, Derek Peter
(86) International application number: GB8800224
(87) International publication number: WO8807532

(56) References cited:
- EP-A- 0 206 497
- US-A- 4 177 348

## Description

This invention relates to antiviral compounds and more particularly to esters and amides of nucleoside derivatives which are active against human immunodeficiency virus (HIV), the retrovirus which causes the disease AIDS.

AIDS is a relatively new disease. It was discovered in 1981 and several thousand cases of the disease have been diagnosed since then. It is anticipated that the number will increase to at least several hundred thousand in the next few years. The situation is especially severe in several Central African countries. AIDS is fatal, and about 40% of all diagnosed cases have ended in death. Of those diagnosed as having AIDS three or more years ago it is estimated that 85% are now dead.

Clinical symptoms are weight loss, chronic diarrhoea, persisting fever and opportunistic infections due to loss of T-cells, thus upsetting the overall balance of the immune system. The patient loses his/her ability to combat otherwise insignificant infections.

Several different methods to combat the infection have been tried. Among the methods tried are stimulation of the immune system and conventional treatment of the (secondary) life-threatening infections. So far the most promising method has been to attack the replication of the HIV-virus. Several different compounds interfering with replication have been tried, e.g. phosphonoformate (Foscarnet), suramin, Evans Blue, 3'-azido-3'-deoxythymidine (AZT) and 2', 3'-dideoxynucleosides.

European Patent Application No. 0196185A, for instance, describes pharmaceutical compositions containing AZT, a known compound which has shown great promise in the treatment of AIDS and AIDS-related complex. It is believed that AZT works by inhibiting reverse transcriptase, a vital enzyme in the life cycle of retroviruses.

Further work has been done on alternative reverse transcriptase inhibitors which might avoid the limitations and drawbacks of AZT, for instance bone marrow suppression or the need for frequent administration of relatively large quantities, and among those suggested have been the 2',3'-dideoxynucleosides.

The synthesis and activity of these compounds have been described (Mitsuya and Broder, Proc. Natl. Acad. Sci. 83, 1911 (1986)) and it was demonstrated that both the 2' and 3' positions must be unsubstituted while the 5'-hydroxy group must be present, presumably to allow in vivo conversion to the corresponding nucleotides. The compounds seem to have lower toxicity and higher potency than AZT; 2',3'-dideoxycytidine is now undergoing clinical trials.

European Patent Application No 0206497A discloses 2',3'-dideoxyribofuranoside derivatives of cytosine or purine bases as antiviral compounds. While there is reference to esters of these compounds as possible metabolic precursors, there is no suggestion that esters would possess any advantageous properties compared with the parent 5'-hydroxy compounds and no esters are specifically named or their synthesis exemplified. There is no reference to any corresponding thymidine compounds or of any nucleoside derivatives having N-acylated amino groups.

We have now found that esterification of the 5'-hydroxy group and/or amidation of amino groups present in the purine or pyrimidine ring can give significant advantages in terms of uptake, overall activity and site of action.

Thus according to one feature of the invention we provide use of compounds of formula (I) 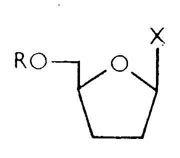
wherein R is a hydrogen atom or a physiologically acceptable acyl group of formula R¹.CO- or R¹.O.CO-, R¹ being an optionally substituted alkyl or aryl group, and X is selected from 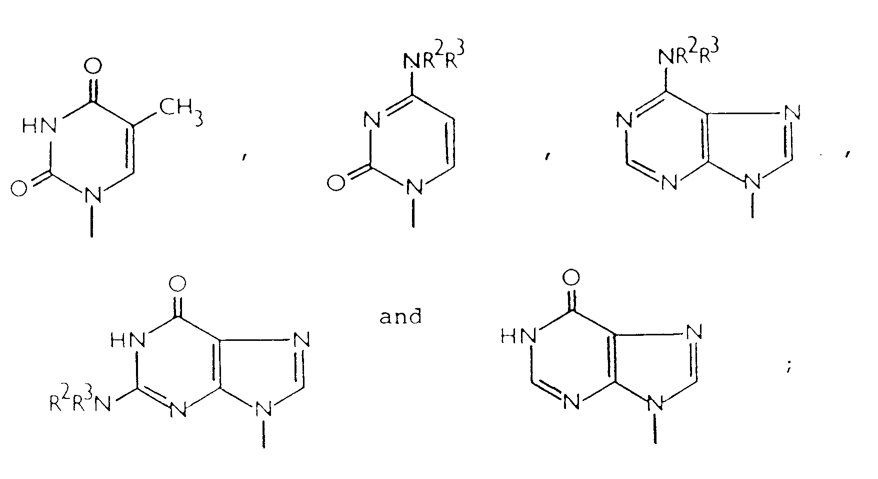
wherein R² and R³, which may be the same or different, are each a hydrogen atom or a physiologically acceptable acyl group of formula R⁴.CO- or R⁴.O.CO-, R⁴ being an optionally substituted alkyl or aryl group, with the proviso that at least one of R and R² must be an acyl group, and/or salts thereof in the preparation of a pharmaceutical composition for combatting neurological disorders caused by neurotropic viruses. X is advantageously a thymine group.

An additional aspect of the invention provides an orally administrable pharmaceutical composition comprising as active ingredient one or more compounds of formula (I) 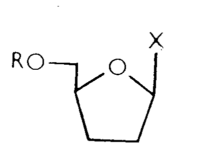
wherein R is a hydrogen atom or a physiologically acceptable acyl group of formula R¹.CO- or R¹.O.CO-, R¹ being an optionally substituted alkyl or aryl group, and X is selected from 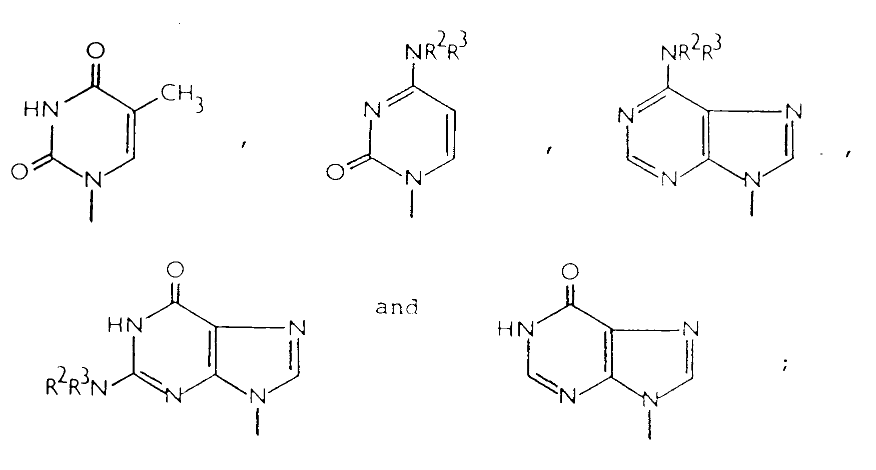
wherein R² and R³, which may be the same or different, are each a hydrogen atom or a physiologically acceptable acyl group of formula R⁴.CO- or R⁴.O.CO-, R⁴ being an optionally substituted alkyl or aryl group, with the proviso that at least one of R and R² must be an acyl group and when R is a hydrogen atom and X is a cytosine moiety in which R² is a group R⁴.CO, then R³ is other than a hydrogen atom, and/or salts thereof.

The compositions may be formulated in conventional manner by admixture of one or more compounds of formula (I) as defined above with excipients and/or carriers.

A yet further aspect of the invention provides novel compounds of formula (I) 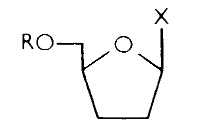
wherein R is a hydrogen atom or a physiologically acceptable acyl group of formula R¹.CO- or R¹.O.CO-, R¹ being an optionally substituted alkyl or aryl group, and X is 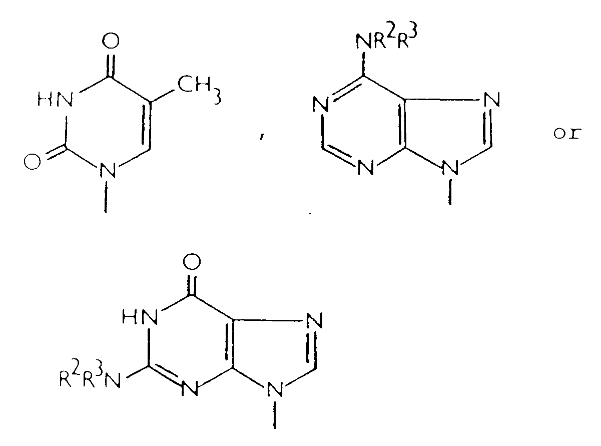
wherein R² and R³, which may be the same or different, are each a hydrogen atom or a physiologically acceptable acyl group of formula R⁴.CO- or R⁴.O.CO-, R⁴ being an optionally substituted alkyl or aryl group, with the proviso that at least one of R and R² must be an acyl group, with the further proviso that when R is an acetyl group then X is not a thymine radical; when R is a benzoyl group then X is not a thymine radical and when R is a 3-(trifluoromethyl)-benzoyl group then X is not an N-unsubstituted adenine radical; and salts thereof.

The acyl groups R, R² and R³ in formula (I) are preferably C₁₋₂₀ acyl groups and more preferably C₂₋₁₈ acyl groups (the term "acyl" as used herein is intended to include groups derived from either carboxylic or carbonic acids). The acyl group may be saturated, unsaturated or contain an aromatic system, and can include, for instance, C₁₋₈ alkanoyl and C₇₋₂₀ aroyl groups. The acyl groups may be substituted, for instance by hydroxy or carboxy groups. Alkanoyl groups can carry C₆₋₁₂ aryl groups. Suitable examples include formyl, acetyl, butyryl, pivaloyl, hexanoyl, stearoyl, palmitoyl, succinoyl, phenylacetyl, benzoyl, isobutyloxycarbonyl, ethyloxycarbonyl and benzyloxycarbonyl groups.

The compositions wherein R² and R³ are hydrogen and R is a group R¹.O.CO- as defined above form one particularly preferred aspect of the invention. Another preferred group of compounds according to the invention are those in which R² is an acyl group as defined above, R³ is hydrogen or an acyl group as defined above and R is hydrogen or an acyl group as defined above. In general R³ is preferably hydrogen.

The salts of the compounds of formula (I) may be acid addition salts with organic or inorganic acids, for instance hydrochloric or phosphoric acids or methanesulphonic acid, ethane disulphonic acid, 2-naphthylsulphonic acid, pivalic acid and pamoic acid. Antiviral counter-ions such as phosphonoformate or suramin may also be used. Organic or inorganic base salts may be formed with acidic groups present in the molecule; suitable counter-ions include alkali metal ions such as sodium and potassium ions, divalent ions such as calcium and zinc ions and organic ions such as tetraalkylammonium and choline or ions derived from meglumine or ethylene-diamine. Salts according to the invention may be formed by reaction of the compound of formula (I) with an appropriate acid or base.

The compositions according to the invention may be used in the treatment and/or prophylaxis of retrovirus infections, in particular HIV infections.

It is believed that the esters of formula (I) are not themselves inhibitors of reverse transcriptase but are converted in vivo to the 5-hydroxy-2,3-dideoxynucleosides. Nevertheless the esterification and/or amidation of the hydroxy and amino groups gives surprising advantages in terms of uptake and sustained activity. The compounds of formula (I) are more lipophilic than the parent compounds and this permits rapid and efficient absorption from the gastro-intestinal tract; the absorption rate may be optimised by careful choice of the acyl group to give the desired balance of lipophilicity and hydrophilicity. The lipophilic nature of the compunds of formula (I) also gives the molecules the ability to penetrate the cell membranes more easily and leads to higher intracellular concentrations, giving an improved dose/effect ratio. The steady hydrolysis of the ester compounds ensures a sustained concentration of the active compound in the cell and thereby permits longer intervals between doses, overcoming a significant drawback of the prior art compounds such as AZT.

Finally, the compounds according to the invention can penetrate the blood-brain barrier and thus permit treatment of the neurological disorders which have been observed to be related to the presence of neurotropic viruses, e.g. retroviruses such as HIV, and lentiviruses (Yarchoan et al, The Lancet, January 17, 1987, page 132). This is a significant advantage compared to the corresponding unsubstituted compounds or other antiviral compounds and is not referred to anywhere in the prior art, for instance in EP-A-0206497. Attempts have been made to treat these neurological disorders with AZT but with limited success.

The known compounds of formula (I) are described in a number of publications; there is, however, no indication that they might be active against HIV.

Compounds of formula (I) and, in particular, the novel compounds defined above, may be prepared by acylation of compounds of formula (II) 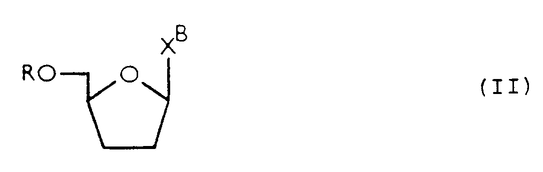
[wherein R is as hereinbefore defined and X^{B} is as hereinbefore defined for X except that R and R² and/or R³ may each additionaly represent a protecting group, with the proviso that at least one of R, R² and R³ is a hydrogen atom] with an acylating agent serving to introduce an acyl group R¹CO-, R¹OCO-, R⁴CO- or R⁴OCO-, followed where required by removal of any protecting groups and/or unwanted acyl substituents.

It should be noted that where, in the starting material, more than one of R, R² and R³ is hydrogen, diacylation or triacylation may occur.

In general, we have found that using acid anhydrides as acylating agents to introduce a group R¹CO or R⁴CO O-acylation takes place more readily than N-acylation whereas using acid halides, N-acylation or even N-diacylation predominates. However, N-acyl groups R⁴CO- may be removed selectively, for example by reaction with a phenol such as p-methylphenol. Where it is desired to ensure that O-acylation to introduce a group R¹OCO- is effected while R² and R³ remain as hydrogen atoms, it may be desirable to protect the exocyclic nitrogen atom first, to form a compound of formula (I) in which R² and R³ are N-protecting groups, these being removed after introduction of the 0-acyl group. Such protecting groups may, in fact, be conventional N-protecting groups including other groups R⁴OCO- which may be selectively removed in the presence of the O-acyl group R¹OCO-. Thus, for example, an N-benzyloxy-carbonyl group may be used to protect an exocylic amino and if the O-acyl group R⁴OCO- is not one which is removable by reduction, for example a straight chain alkoxycarbonyl group, the N-benzyloxy-carbonyl group can readily be removed selectively using hydrogen and a noble metal catalyst such as palladium.

In general, where more than one of R, R² and R³ are hydrogen, a mixture of acylated compounds may be produced. However, the individual components may readily be separated, for example by chromatography.

Suitable acylating agents for use in the reaction have the formula Ac-L where L is a leaving group. When the acyl group Ac- is derived from a carboxylic acid, i.e. is of formula R¹-CO- or R⁴-CO-, then suitable acylating agents include the acid halides and acid anhydrides advantageously in the presence of a base; when the acyl group is derived from a carbonic acid, i.e. is of formula R¹.O.CO- or R⁴.O.CO-, then acylating agents include the haloformate esters and reactive carbonic acid diesters. The base for use in the reaction with the acid halide or anhydride may, for example, be a heterocyclic base such as pyridine or dimethylamino-pyridine. The latter increases the speed of the reaction and may be used advantageously with pyridine. The reaction will normally be carried out in the presence of an inert solvent such as dimethyl-formamide or a halogenated hydrocarbon such as dichloromethane.

The starting compounds of formula (II) wherein R, R² and R³ are all hydrogen atoms are well described in the literature - see, for instance, Lin et al, J. Med. Chem. 30, 440 (1987).

The pharmaceutical compositions according to the invention may be formulated conventionally by means well known in the art, and may be administered by any convenient route, for instance orally, rectally, vaginally, intraveneously or intramuscularly. Examples of suitable formulations include tablets and capsules, aqueous formulations for intravenous injection and oil-based formulations for intramuscular injection. Suitable dosages will lie in the range 0.1 to 100mg per kilogram of bodyweight per 24 hour period. The compositions according to the invention may also contain other active antivirals for instance acyclovir, phosphonoformate, suramin, Evans Blue, interferons or AZT.

The invention is illustrated by the following Examples. Capsugel is a Trade Mark.

### Example 1

### 2',3'-Dideoxy-5'-0-palmitoyl-cytidine

Palmitoyl chloride (2.80g, 10.2 mmol) is added dropwise during 30 minutes to a stirred solution of 2',3'-dideoxycytidine (2.11g, 10 mmol) in dry 1:1 pyridine/N,N-dimethylformamide (130ml) at 0°C. The mixture is stirred for 30 hours. Water (20ml) is added and the mixture is evaporated. The product is purified on a column of silica gel with methanol/chloroform/hexane as solvent.

### Example 2

### 5'-0-Butyryl-2',3'-dideoxy-adenosine

Butyryl chloride (1.09g, 10.2mmol) is added dropwise during 30 minutes to a stirred solution of 2',3'-dideoxyadenosine (2.45g, 10 mmol) in dry 1:1 pyridine/N,N dimethylformamide (100ml) at 0°C. The mixture is stirred at 0°C for 30 hours, water (20ml) is added and the mixture is evaporated. The product is purified on a column of silica gel with methanol/chloroform as solvent.

### Example 3

### 3'-Deoxy-5'-0-hexanoyl-thymidine

3'-Deoxythymidine (0.0100 g, 4.4203 x 10⁻⁵mole) was dissolved in a mixture of pyridine (0.44ml) and dimethylformamide (0.44 ml) (both distilled from calcium hydride) and cooled to 0°C. Hexanoyl chloride (freshly distilled, 0.00682 ml, 4.8622 x 10⁻⁵ mole) was added with a syringe. The mixture was stirred for 48 hours under nitrogen at 0°C, when thin layer chromatography showed partial conversion. N,N-Dimethyl-4-aminopyridine (0.0001 g) was added under exclusion of air and the mixture was stirred for a further 24 hours when hexanoyl chloride (0.00682 ml, 4.8622 x 10⁻⁵mole) was added. After a further 24 hours water (2 ml) was added and the solution was evaporated under high vacuum. Water was added four times (4 x 2 ml) with high vacuum evaporation between each addition. The resulting semi-solid was dissolved in chloroform and applied to a silica column (E. Merck 9385) and eluted with chloroform and chloroform: ethanol 99:1. The title compound eluted first. Yield 0.0085 g (59.3%), mp 94-96 °C (uncorrected).
¹H NMR(CDCl₃ 300 MHz) δ : 0.90 (t, 3H, J 6.8 Hz), 1.32(m, 4H), 1.66(m,2H), 1.83(m,1H), 1.95 (s,3H), 2.05(m, 2H), 2.37(t, 2H, J 7.5 Hz), 2.45(m, 1H), 4.33(m, 3H), 6.08(d d, 1H, J₁ 4.4 Hz, J₂ 6.70 Hz), 7.40(s, 1H), 8.54(bs, 1H).
¹³C NMR (CDCl₃ 75 MHz) δ : 12.674, 13.879, 22.288, 24.593, 25.919, 31.285, 32.223, 34.183, 64.801, 78.462, 86.180, 110.508, 135.272, 150.163, 163.530, 173.442.

### Example 4

### 3'-Deoxy-5'-0-palmitoyl-thymidine

3'-Deoxythymidine (0.0100 g 4.4203 x 10⁻⁵mole) was dissolved in a mixture of pyridine (0.221 ml) and dimethylformamide (0.221 ml) (both distilled from calcium hydride) and cooled to 0°C. Palmitoyl chloride (freshly distilled, 0.01476 ml, 4.8623 x 10⁻⁵mol) was added with a syringe. The mixture was stirred for 4 days under nitrogen, when thin layer chromatography showed partial conversion. Pyridine (0.221 ml) and dimethylformamide (0.221 ml) (both cooled to 0°C) were added and the resulting mixture stirred at 10°C for 24 hours, when water (2ml) was added. The resulting mixture was evaporated at low temperature under high vacuum. Water was added four more times (4 x 2 ml), with high vacuum evaporation between each addition. The resulting semisolid was suspended in chloroform and applied to a silica column (E. Merck 9385) and eluted first with chloroform, then with chloroform:methanol 9:1. The title compound eluted first. Yield 0.0076g (34.7%) mp 92-94 °C (uncorrected.). ¹H NMR(CDCl₃ 300 MHz) δ : 0.88(t 3H, J 7.1 Hz), 1.25(m+s 20H), 1.61(m 2H), 1.83(m 1H), 1.95(s 3H), 2.04(m 2H), 2.37(t 2H,J 3 Hz), 2.42(m 1H), 4.32(m 3H), 6.07(dd 1H), 7.40(s 1H), 8.20(broad s, 1H).
¹³C NMR(CDCl₃ 75 MHz) δ : 12.68, 14.12, 22.69, 24.91, 25.89, 29.15, 29.25, 29.36, 24.46, 29.60, 29.68(large peak - 5 carbon atoms), 31.93, 32.23, 78.47, 86.16, 110.48, 135.25, 150.03, 163.35, 173.48.

### Example 5

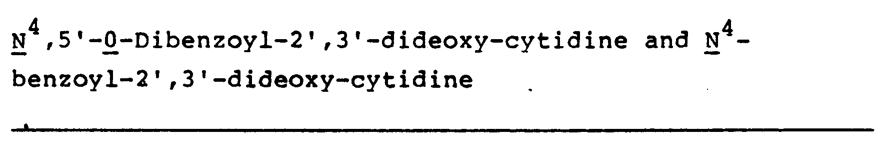

2',3'-Dideoxy cytidine (0.0200 g, 9.469x10⁻⁵ mole) and N,N-dimethylaminopyridine (0.0127g, 10.367x10⁻⁵ mole) were dissolved in dichloromethane (1.0 ml, distilled from calcium hydride). Benzoyl chloride (0.0146g, 10.367x10⁻⁵ mole) was added with a syringe. The resulting mixture was stirred for 24 hours before distilled water (2.0 ml) was added. After complete evaporation (high vacuum) the residue was chromatographed on a silica column with chloroform and chloroform:ethanol 9:1. N⁴,5'-0-Dibenzoyl-2',3'-dideoxy-cytidine eluted first, followed by N⁴-benzoyl-2',3'-dideoxy-cytidine.
N⁴,5'-0-Dibenzoyl-2',3'-dideoxy-cytidine
Yield 0.0144 g (36.4%) M.p. 180-190°C (uncorrected)(not recrystallized). ¹HNMR(CDCl₃, 300 MHz) δ : 1.76-1.92 (m, 1H), 2.04-2.16 (m, 1H), 2.18-2.30(m, 1H), 2.54-2.70(m, 1H), 4.47-4.56(m, 1H, H4'), 4.56 (broad d, 2H, H5'), 6.10(dd, 1H,H1'), 7.43(d, 1H,H5), 7.46-7.54(broad t, 4H,Ph), 7.56-7.64 (broad t, 2H, Ph), 7.86 (broad d, 2H,Ph), 8.05 (broad d, 2H,Ph), 8.26 (d, 1H, H6, J 7.46 Hz), 8.59 (broad, 1H, NH). ¹³C NMR(CDCl₃, 75 MHz) δ : 25.03, 33.42, 64.73, 80.16, 88.27, 95.90, 127.42, 128.69, 129.06, 129.36, 129.57, 133.16, 133.16, 133.64, 144.19, 162.06, 166.27.
N⁴ benzoyl-2',3'-dideoxy-cytidine
Yield 0.0060 g (28.0%) M.p. 202-205°C (uncorrected) (not recrystallized). ¹HNMR(CDCl₃, 300 MHz) δ : 1.85-2.05(m, 2H), 2.16-2.30(m, 1H), 3.78-3.88 and 4.06-4.16(ABX, 2H, H5'), 4.29(m, 1H, H4'), 6.12(dd, 1H,H1'), 7.41-7.64(m, 3H,Ph), 7.92 (broad d, 2H, Ph), 8.51(d, H6), 8.52 (broad, 1H, NH).
¹NMR(DMSOd₆; 300 MHz) δ : 1.72-1.90 (m, 2H), 1.90-2.10(m, 1H), 2.35-2.48(m, 1H), 3.55-3.65 and 3.72-3.82 (2H, ABX, H5'), 4.12 (m, 1H, H4'), 5.16(t, 1H, OH), 5.95(dd, 1H, H1'), 7.33(d, 1H, H5), 7.47-7.56(broad t, 2H, Ph), 7.59-7.66 (broad t, 2H, Ph), 7.59-7.66(broad t, 1H, Ph), 7.99(broad d, 2H, Ph), 8.55(d, 1H, H6 J 7.38 Hz), 11.22(s, 1H, NH). ¹³C NMR(CDCl₃ + 5% DMSOd₆, 75 MHz) δ : 23.66, 33.37, 62.00, 82.96, 87.82, 95.76, 127.53, 128.53, 132.34, 132.66, 132.95, 145.30, 154.91, 162.19.

### Example 6

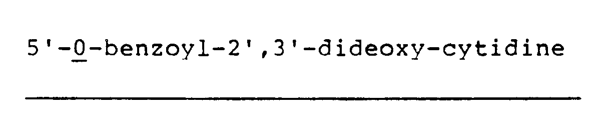

N⁴,5'-0-Dibenzoyl-2',3'-dideoxy-cytidine (0.0142g, 3.385x10⁻⁵ mole) and p-methylphenol (0.0183 g, 1.689x10⁻⁴ mole) were dissolved in toluene (0.5ml distilled from sodium and benzophenone) and stirred at room temperature for 24 hours. The temperature was then increased to 120°C and the mixture was stirred for a further 12 hours. At this time thin layer chromatography (silica, chloroform:ethanol 99:1 and 9:1) revealed almost complete consumption of the starting material. The toluene was evaporated and the residue chromatographed on silica with chloroform, chloroform:ethanol 99:1 and chloroform:ethanol 9:1. The compounds were eluted in the following order: p-methylphenol, N⁴,5'-0-dibenzoyl-2',3'-dideoxy-cytidine and 5'-0-benzoyl-2',3'-dideoxy-cytidine. Recovered N⁴,5'-0-dibenzoyl-2',3'-dideoxy-cytidine 0.0018 g (13 %).
Yield (5'-0-benzoyl-2',3'-dideoxy-cytidine) 0.0092g (86.0%). Glassy material. M.p. 114-116°C (uncorrected). (not recrystallized) ¹HNMR(CDCl₃, 300 MHz) δ : 1.67-1.86 (m, 1H), 2.02-2.21(m,2H),2.44-2.62(m,1H),4.41-4.46 (m,1H,H4'), 4.52-4.68 (ABX,2H,H5'), 5.54(d, H5, J 7.2 Hz), 6.08(dd, H1'), 7.44-7.50 (broad t, 2H Ph), 7.57-7.64 (broad d, 1H, Ph), 7.81(d, H6, J 7.2 Hz), 8.04(broad d 2H, Ph), 5.1-6.3 (very broad, 2H, NH₂). ¹³C NMR(CDCl₃, 75 MHz) δ : 25.51, 33.28, 65.28, 73.98, 79.25, 87.64, 93.07, 128.55, 129.57, 129.63, 133.41, 140.93, 155.77, 164.46.

### Example 7

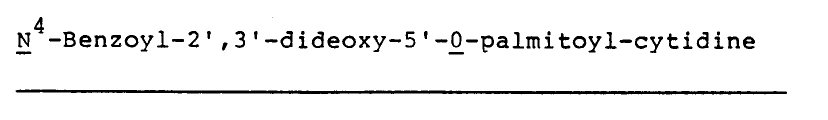

N⁴-Benzoyl-2',3'-dideoxycytidine (0.0215 g, 6.797x10⁻⁵ mole) was dissolved in a mixture of pyridine (0.25 ml) and dimethylformamide (0.25 ml). N,N-dimethylaminopyridine (0.0083 g, 6.797x10⁻⁵ mole) and palmitoyl chloride (0.0374 g, 1.359x10⁻⁴ mole) were added at room temperature. The resulting mixture was heated to 60°C and stirred at this temperature for 12 hours, when a new aliquot of palmitoyl chloride (0.0374 g, 1.359x10⁻⁴ mol) was added at room temperature. The resulting mixture was heated to 60°C and stirred at this temperature for 12 hours, when a new aliquot of palmitoyl chloride (0.0374g, 1.359x10⁻⁴mol) and pyridine (0.25 ml) were added at room temperature. The temperature was again raised to 60°C and kept there for a further 8 hours. Water (2 ml) was added and the solvents removed at high vacuum. The resulting semi-solid was applied to a silica column and eluted with chloroform and chloroform:ethanol 99:1. The product was isolated as a white powder contaminated with palmitic acid. No attempt was made to remove the palmitic acid at this stage. Yield (after subtracting excess palmitic acid from the ¹HNMR-integration): 0.0199 g (52.8 %).
¹HNMR(CDCl₃, 300 MHz)δ : 1.95(t, CH₃), 1.2-1.6 (m, CH₂-alkyl), 2.06-2.20(m, 1H), 2.25-2.35(m, 1H), 2.35-2.50(m,4H), 2.60-2.75 (m,1H), 4.40-4.58(m, 3H, H4' and H5'), 6.15(dd, H1'), 7.55-7.80(m, 4H, Ph+H5), 8.05 (broad d, 2H, Ph), 8.26(d, 1H,H6).
¹³C NMR(CDCl₃, 75 MHz) (sample containing free palmitic acid)δ : 14.12, 22.69, 24.71, 24.95, 29.09, 29.17, 29.27, 29.36, 29.36, 29.45, 29.48, 29.60, 29.68, (broad-large resonance-several carbon atoms), 31.92, 33.29, 34.06, 34.22, 64.22, 80.13, 88.43, 96.07, 128.15, 128.76, 132.86, 133.13, 144.80, 163.01, 173.43, 179.60.

### Example 8

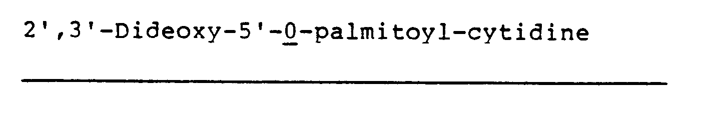

N⁴-benzoyl-2',3'-dideoxy-5'-0-palmitoyl-cytidine (0.0199 g, 3.587x10⁻⁵ mole) (contaminated by some palmitic acid) and p-methylphenol (0.0256 g, 2.367x10⁻⁴ mole) were dissolved in toluene (0.5 ml, distilled from sodium and benzophenone). The resulting solution was refluxed for 15 hours. The toluene was evaporated and the residue chromatographed on a silica column and eluted with chloroform, chloroform:ethanol 99:1 and chloroform:ethanol 9:1. The benzoate of the p-methylphenol and the palmitic acid contamination from the preceding step were eluted first followed by p-methylphenol, N⁴-benzoyl-2',3'-dideoxy-5'-0-palmitoyl-cytidine and 2',3'-dideoxy-5'-0-palmitoyl-cytidine. Yield (2',3'-dideoxy-5'-0-palmitoyl-cytidine )0.0107 g (66.2 %) M.p. 120-122 °C (uncorrected) (not recrystallized).
¹HNMR(CDCl₃, 300 MHz) δ : 0.88 (t, CH₃), 1.2-1.38 (broad s, 22H, alkyl chain), 1.57-1.76(m, 4H), 1.96-2.06(m, 1H), 2.06-2.18(m, 1H), 2.35(t, CH₂-COO), 2.43-2.58(m, 1H), 4.32-4.40(m, 3H, H5'+H4'), 5.0-6.0 (very broad 2H, NH₂), 5.67 (d, 1H, H5, J 7.51 Hz), 6.05(dd, H1'), 7.79(d, H6, J 7.51 Hz).
¹³C NMR(CDCl₃, 75 MHz) δ : 14.13, 22.69, 24.91, 25.50, 29.16, 29.27, 29.36, 29.47, 29.61, 29.65 and 29.69 (these two resonances represent several carbon atoms) 31.92, 33.16, 34.21, 64.81, 73.99, 79.18, 87.71, 92.82, 96.89, 141.09, 155.74, 165.40, 173.49.

### Example 9

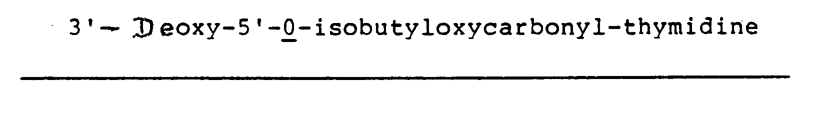

3'-Deoxythymidine (0.0100 g, 4.42.10⁻⁵ mole) and N,N-dimethylaminopyridine (0.0059 g, 4.8x10⁻⁴ mole) were suspended in dry dichloromethane (1ml) and cooled to 0°C. Isobutyl chloroformate (12.62 ul, 8.84x10⁻⁵ mole) was added. The resulting mixture was stirred at room temperature for 11 days. Water (2 ml) was added. After complete evaporation at high vacuum, the residue was chromatographed on a silica column. The product was eluted with chloroform and chloroform:ethanol = 99:1
Yield 0.0119 g (82.4%), mp 128-130 °C (uncorrected) (not recrystallised).
¹HNMR (CDCl₃; 300 MHz) δ : 0.96(d, 6H, J 6.75 Hz), 1.95(s, 3H), 1.91-2.18(m, 4H), 2.4(m, 1H), 3.97(d, 2H, J 6.59 Hz), 4.32(m, 1H), 4.40(ABX, 2H), 6.12(q, 1H), 7.56(s, 1H), 8.47(broad s, 1H).
¹³CNMR(CDCl₃, 75 MHz) δ : 12.51, 18.89,(2 carbon atoms), 25.40, 27.81, 32.46, 67.73, 74.61, 78.41, 85.97, 110.58, 135.64, 150.22, 155.21, 163.60. MSCI (isobutane): 327(M+1, 41.4), 209(5.3), 202(7.4), 200(67.0), 169(16.5), 167(18.1), 145(58.4), 127(100), 83(24.6).

### Example 10

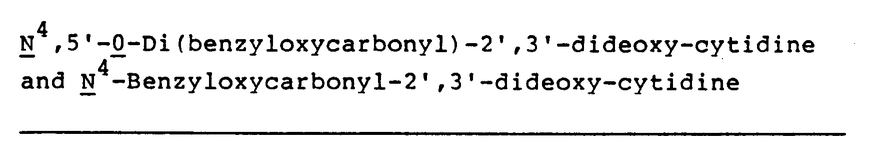

2',3'-Dideoxy-cytidine (0.0250 g, 1.178 x 10⁻⁴ mole) was dissolved in a mixture of pyridine (0.25 ml) and N,N-dimethylformamide (0.25 ml) and cooled to 0 °C. Benzyl chloroformate (0.0603 g, 3.534 x 10⁻⁴ mole) was added with a syringe. N,N-dimethylaminopyridine (0.0144 g, 1.178 x 10⁻⁴ mole) was added and the resulting solution stirred at room temperature for 12 hours. Thin layer chromatography (silica, chloroform:ethanol 9:1) indicated partial conversion at this point. The mixture was cooled to 0°C and benzyl chloroformate (0.0603 g, 3.534 x 10⁻⁴ mole) was added with a syringe. The mixture was stirred for a further 24 hours at room temperature. Water (2 ml) was then added and the solution was evaporated at high vacuum. The resulting semi-solid was applied to a silica column and eluted with chloroform and chloroform: ethanol 99:1.
N⁴-Benzyloxycarbonyl-2',3'-dideoxy-cytidine
Yield 0.0385 g (84.9 %). Glassy material. ¹HNMR (CDCl₃, 300 MHz)δ : 1.82-1.98 (m, 2H), 2.10-2.22 (m, 1H), 2.42-2.59(m, 1H), 3.05 (broad, 1H, OH), 3.76 and 3.80 (ABX, 2H, H5'), 4.24 (m, H4'), 5.17 (s, 2H, 0-CH₂-Ph), 6.06 (dd, 1H,H1'), 7.24 (d, 1H, H5, J 7.57 Hz) 7.93 (broad, 1H, NH), 8.50 (d, 1H, H, J 7.57 Hz) ¹³CNMR (CDCl₃, 75 MHz)δ : 24.10, 33.37, 62.93, 67.85, 82.72, 88.19, 94.26, 128.33, 128.44, 128.64, 134.94, 145.01, 152.28, 155.23, 162.11.
N⁴,5'-0-di(benzyloxycarbonyl)-2',3'-dideoxy-cytidine was also isolated in small quantities. This product coeluted with several contaminants and decomposition products. The product was finally isolated by careful rechromatography on a silica column with pure chloroform as eluent.
N⁴,5'-0-di(benzyloxycarbonyl)-2',3'-dideoxy-cytidine.
Yield: 0.0075 g (13.2%). Glassy material. ¹HNMR(CDCl₃, 300MHz) δ : 1.64 - 1.82 (m, 1H), 1.92-2.08 (m, 1H), 2.08-2.22 (m, 1H), 2.46-2.62 (m, 1H), 4.32-4.40 (m, 1H, H5'), 4.34-4.52 (ABX, 2H, H4'), 5.21 (s, 2H, CH₂-0), 5.23 (s, 2H, CH₂-0), 6.06 (dd, 1H,H1'), 7.21 (d, H5, J 7.38 Hz), 7.39 (broad, 10H, 2Ph), 7.5 (broad, 1H, NH), 8.16 (d, 1H, H6, J 7.38 Hz). ¹³C NMR(CDCl₃, 75 MHz) δ : 24.83, 33.23, 67.67, 67.95, 70.06, 79.51, 88.10, 94.16, 128.36, 128.52, 128.71, 134.86, 144.05, 152.12, 154.93, 162.05.

### Example 11

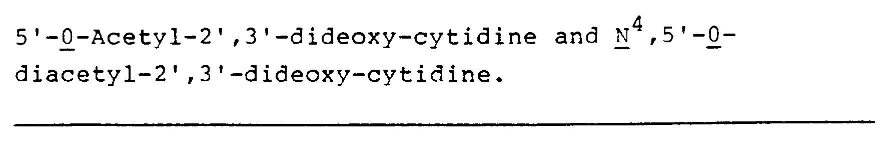

2',3'-dideoxy-cytidine (0.0300 g, 1.42x10⁻⁴ mole) and N,N-dimethylaminopyridine (0.0087 g, 7.10x10⁻⁵ mole) were dissolved in a mixture of dichloromethane (1 ml) and pyridine (1 ml). The resulting solution was cooled to 0°C and acetic anhydride (0.0290 g, 2.84x10⁻⁴ mole) was added with a syringe. The reaction mixture was stirred at room temperature for 24 hours. Water (4 ml) was then added and the solvents were removed by high vacuum evaporation. The resulting solid was chromatographed on a silica column and eluted with chloroform:ethanol 99:1, chloroform:ethanol 9:1 and chloroform:ethanol 7:3.
5'-0-acetyl-2',3'-dideoxy-cytidine
Yield 0.0120 g (31.3 %) Oil, glassy material ¹HNMR(CDCl₃, 300 MHz)δ : 1.60-1.78(m, 1H), 1.94-2.20(m, 2H), 2.12(s, 3H), 2.40-2.58(m, 1H), 4.32(m, 3H, H4'+H5'), 5.77(d, 1H, H5, J 7.20 Hz), 6.05(dd, 1H, H1'), 7.40(d, 1H, H6, J 7.20 Hz), 5.0-7.3(very broad, 2H, NH₂).
¹³CNMR (CDCl₃, 75 MHz, pulse delay 3s)δ : 20.85, 25.54, 33.02, 65.04, 78.98, 87.54, 93.58, 140.61, 155.76, 165.63, 170.63.
N⁴,5'-0-diacetyl-2',3'-dideoxy-cytidine
Yield 0.0268 g (63.9%) M.p. 230°C (uncorrected) (not recrystallized).
¹HNMR (CDCl₃, 300 MHz)δ : 1.63-1.80(m, 1H), 1.96-2.09(m, 1H), 2.10-2.23(m, 1H), 2.15(s, 3H), 2.30(s, 3H), 2.48(m, 1H), 4.30-4.45(m, 3H), 6.06(dd, 1H, H1'), 7.46(d, 1H, H5 J 7.54 Hz), 8.19(d, 1H, H6, J 7.54 Hz), NH not seen. ¹³CNMR (CDCl₃, 75 MHz, pulse delay 3s)δ : 20.84, 24.85, 33.21, 64.40, 79.91, 88.20, 96.03, 143.96, 155.04, 162.90, 170.49, 171.12.

### Example 12

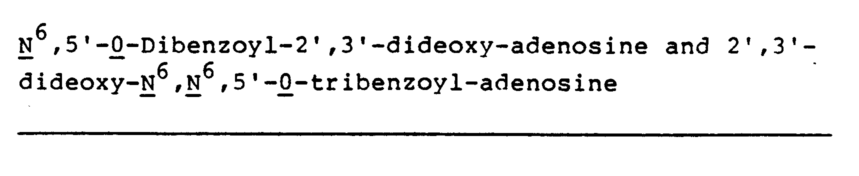

2',3'-Dideoxyadenosine (0.0250 g, 1.063x10⁻⁴ mole) was dissolved in a mixture of dichloromethane (1.0 ml) and pyridine (0.25 ml) and cooled to 0°C. Benzoyl chloride (0.0299 g, 2.125x10⁻⁴ mole) was added with a syringe and the temperature raised to room temperature. The mixture was stirred for 24 hours, recooled to 0°C and benzoyl chloride (0.0299 g, 2.125x10⁻⁴ mole) was added for the second time. The reaction mixture was stirred for a further 12 hours at room temperature. Water (4ml) was added and solvents and water were removed by high vacuum evaporation. The resulting semi-solid was chromatographed on a silica column and eluted with chloroform and chloroform:ethanol 99:1. Not all fractions contained pure compounds after the first column. The impure fractions were chromatographed a second time on a silica column and eluted with chloroform and chloroform: ethanol 99:1.
N⁶,5'-0-Dibenzoyl-2',3'-dideoxy-adenosine
Yield: 0.0387 g (82%). Colorless oil. ¹HNMR(CDCl₃, 300 MHz)δ : 2.17-2.37(m, 2H), 2.57-2.71(m,1H), 2.73(m, 1H), 4.48-4.68(ABX+m, 3H, H5'+H4'), 6.37(dd, 1H, H1') 7.39-7.66 (complex pattern, 6H, 2Ph), 7.87-8.06 (complex pattern 4H, 2Ph), 8.26(s,1H), 8.79(s,1H), 8.99(broad s, 1H, NH). ¹³C NMR(CDCl₃, 75 MHz, pulse delay 3s)δ : 26.39, 32.34, 65.51, 79.57, 86.23, 127.79, 128.48, 128.88, 129.49, 129.59, 132.77, 133.68, 141.38, 149.40, 151.05, 152.58, 164.46, 166.30.
2',3'-Dideoxy-N⁶,N⁶,5'-0-tribenzoyl-adenosine
Yield: 0.0087 g (15%) Clear glassy material. ¹HNMR(CDCl₃ 300 MHz)δ : 2.14-2.34(m, 2H), 2.56-2.77(m, 2H), 4.52-4.63(m, 3H, H4'+H5'), 6.36(dd, 1H,H1'), 7.32-7.58(complex pattern, 9H, 3Ph), 7.83-7.89(dd, 4H, 2Ph), 7.98-8.02(dd, 2H, 1Ph), 8.33(s, 1H), 8.62(s, 1H). ¹³CNMR(CDCl₃, 75 MHz, pulse delay 3s)δ : 26.13, 32.37, 65.61, 79.56, 86.18, 128.05, 128.51, 128.71, 129.44, 129.66, 132.96, 133.30, 134.03, 143.29, 151.73, 152.03, 152.29, 166.33, 172.28.

### Example 13

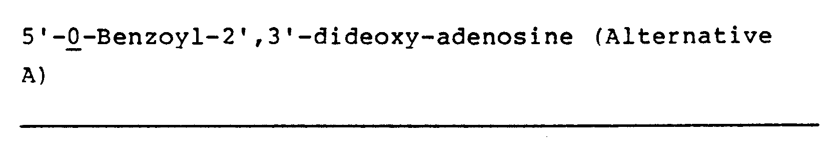

2',3'-Dideoxy-N⁶,N⁶,5'-0-tribenzoyl-adenosine (0.0294 g, 5.369x10⁻⁵ mole) and p-methylphenol (0.0290 g, 2.685x10⁻⁴ mole) were dissolved in toluene (1.0 ml distiled from sodium and benzophenone) and stirred at 50 °C for 1 hour. The temperature was then raised to 110°C and kept there for 24 hours. (The conversion from 2',3'-dideoxy-N⁶,N⁶,5'-0-tribenzoyl-2',3'dideoxy-adenosine was fast (TLC) and the conversion from N⁶,5'-0-dibenzoyl-2',3'-dideoxyadenosine to 5'-0-benzoyl-2',3' -dideoxy-adenosine was slow (TLC)). The toluene was evaporated and the residue chromatographed on a silica column with chloroform, chloroform:ethanol 99:1 and chloroform: ethanol 9:1.
Yield 0.0079 g (43.3 %). Oil, which form foams upon vacuum drying. ¹HNMR(CDCl₃, 300 MHz) δ : 2.14-2.32(m, 2H), 2.52-2.64(m, 1H), 2.65-2.77(m, 1H), 4.50-4.66(m, 3H, H4' and H5'), 5.66(broad s, 1H, NH), 6.31(dd, 1H, H1'), 7.40-7.47(m, 2H, Ph), 7.53-7.61(m, 1H, Ph), 7.96-8.02(m, 2H, Ph) 8.05(s, 1H), 8.34(s, 1H).
¹³CNMR (CDCl₃, 75 MHz, pulse delay 3s) δ : 26.40, 32.38, 65.60, 79.29, 85.84, 120.29, 128.46, 129.55, 129.62, 133.26, 138.80, 149.28, 152.95, 155.34, 166.35. 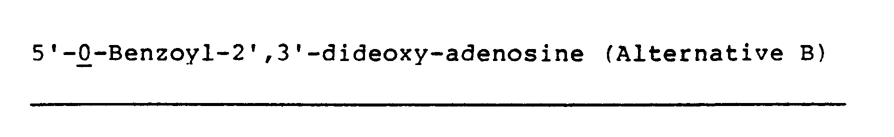
N⁶, 5'-0-Dibenzoyl-2',3'-dideoxy-adenosine (0.0200 g, 4.510x10⁻⁵ mole) and p-methylphenol (0.0122 g, 1.127x10⁻⁴ mole) were dissolved in toluene (1.0 ml distilled from sodium and benzophenone) and stirred at 50 °C for 1 hour. The temperature was then raised to 110°C and kept there for 24 hours. The toluene was evaporated and the residue chromatographed on a silica column with chloroform, chloroform:ethanol 99:1 and chloroform: ethanol 9:1
Yield 0.0064 g (41.8%). (¹HNMR- and ¹³CNMR spectral data were identical with those obtained from the reaction of 2',3'-dideoxy-N⁶,N⁶,5'-0-tribenzoyl-adenosine with p-methylphenol).

### Example 14

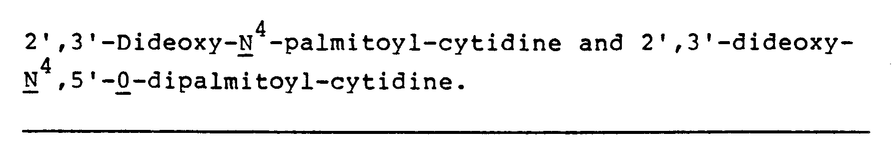

2',3'-Dideoxycytidine (0.005 g, 2.356x10⁻⁵ mole) was dissolved in a mixture of pyridine (0.22 ml) and dimethylformamide (0.22 ml) and cooled to 0°C. Palmitoyl chloride (8 1, 2.59x10⁻⁵ mole) was added with a syringe. Precipitates were formed immediately. To increase the solubility more pyridine (0.22 ml) was added. After 48 hours of stirring the temperature was increased to 15°C. After 24 more hours at this temperature palmitoyl chloride (10 »l, 3.24x10⁻⁵ mole) and N,N-dimethylaminopyridine (cat. amt.) were added. The reaction mixture was stirred for 4 days at 0°C. Water (2 ml) was added and the solution was evaporated under high vacuum. Water was added four more times (4x2 ml) with complete evaporation after each addition. The products were isolated by flash chromatography on silica gel eluted with chloroform and subsequently with chloroform:ethanol 9:1.
2',3'-Dideoxy-N⁴-palmitoyl-cytidine
Yield: 0.0032 g (30 %) white powder. ¹HNMR(CDCl₃ 200 MHz)δ: 0.87(t, 6H, 2xCH₃), 1.21-1.40(broad, 24H), 1.44-1.80(broad, 4H), 1.80-2.00(m, 2H), 2.10-2.25(m, 1H), 2.30-2.42(t, 4H), 2.43-2.60(m, 1H), 3.81 and 4.07(dxAB, 2H H5') 4.20-4.27(m, 1H, H4'), 6.07(dd, H1'), 7.40(H5), 8.15-8.25(broad, 1H,NH). 8.37(d, H6, J 7.32 Hz).
2',3'-Dideoxy-N⁴,5'-0-dipalmitoyl-cytidine
Yield: 0.0049 g (30 %) white powder

### Example 15

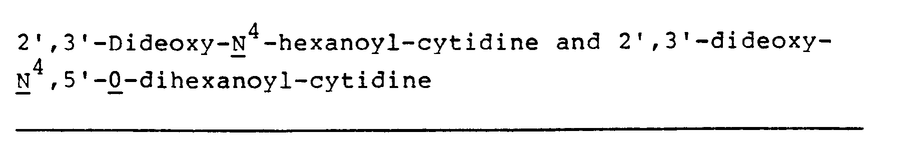

2',3'-Dideoxycytidine (0.0050 g, 2.356x10⁻⁵ mole) was dissolved in a mixture of pyridine (0.22 ml) and dimethylformamide (0.22 ml) and cooled to 0°C. Hexanoyl chloride (3.7 »l, 2.60x10⁻⁵ mole) was added with a syringe. The resulting mixture was stirred at 0°C for 48 hours.
The temperature was increased to 15°C and the mixture stirred for 24 more hours when hexanoyl chloride (3.7 »l) and N,N-dimethylaminopyridine (cat. amt.) were added. The resulting solution was stirred at 0°C for 5 days. The solvents were then evaporated at high vacuum. Water was added four times (4x2 ml) with complete evaporation after each addition. The products were isolated by chromatography on a silica column eluted with chloroform and chloroform: ethanol 9:1.
2',3'-Dideoxy-N⁴-hexanoyl-cytidine
Yield: 0.0018 g (24 %) white powder. ¹H NMR(CDCl₃, 200 MHz)δ : 0.88(t 3H), 1.15-1.40(m, 4H), 1.55-1.75(m, 2H), 1.85-1.98(m, 2H), 2.10-2.25(m, 2H), 2.41(t, 2H), 2.4-2.6(m, 2H), 3.93(dxAB, J AH4' 2.62 Hz, J BH4' 3.92 Hz, JAB 12.00 Hz, 2H), 4.25(m, 1H, H4'), 6.06(dd, 1H, H1'), 7.41(broad d, 1H, H5'), MSCI(isobutane): 310(M+1, 2.6), 252(3.3), 250(4.0), 248(2.5), 212(4.8), 211(12.5), 210(100.0), 201(3.1), 199(4.3), 154(2.7), 153(9.8), 152(5.5), 138(2.9), 116(2.4), 113(3.6), 112(24.6), 109(2.6), 101(35.9), 85(4.3), 83(9.0).
2',3'-Dideoxy-N⁴-5'-0-dihexanoyl-cytidine
Yield: 0.0031 g (32 %) white powder. ¹H NMR(CDCl₃, 200 MHz)δ : 0.89(broad t, 6H, 2-CH₃), 1.2-1.4(m, 10H), 1.5-1.85 (m, 5H), 1.85-2.10(m, 1H), 2.10-2.25(m, 1H), 2.30-2.50(t, 4H, 2xCH₂-CO), 2.45-2.65(m, 1H), 4.25-4.50(m, 3H, H4'+H5'), 6.05(d, H1'), 8.18(d, 1H, H6), 8.0-8.5(broad, 1H, NH). MSCI(isobutane): 408(M+1, 3.5), 311(1.0), 310(2.3), 247(1.0), 245(2.9), 233(1.2), 211(3.7), 210(11.1), 200(12.0), 199(100), 148(2.5), 147(22.4), 117(3.2), 112(7.6), 99(9.5), 83(17.9), 88(17.0), 81(6).

### Example 16

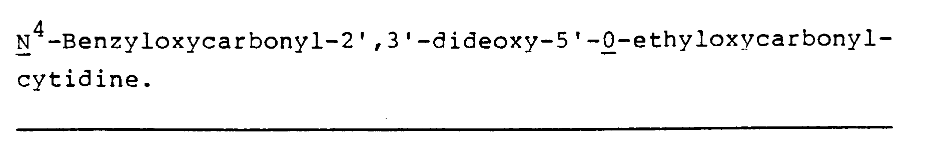

N⁴-Benzyloxycarbonyl-2',3'-dideoxycytidine (0.0358 g, 1.037x10⁻⁴ mole) was dissolved in tetrahydrofuran (1.0 ml, distilled from sodium and benzophenone) and cooled to -78°C. Sodium hydride (0.0045 g 80 % in oil, 1.05x10⁻⁴ mole) was added, and the mixture was allowed to reach room temperature. The reaction mixture was recooled to 0°C when the hydrogen gas evolution ceased. Ethyl chloroformate (0.0111 ml, 1.1403x10⁻⁴ mole (98%)) was added and the reaction was stirred at room temperature for 6 hours. Ethyl chloroformate (0.0111 ml, 1.1403x10⁻⁴ mole) was added once more and the stirring continued for 4 more hours. Saturated ammonium chloride (1ml) was added and the whole mixture evaporated at high vacuum. The resulting solid (including NH₄Cl) was loaded on a silica column and the product eluted with chloroform:ethanol 99:1 and chloroform:ethanol 9:1.
Yield: 0.0350 g (80.9 %). Oil. ¹HNMR(CDCl₃ 300 MHz)δ : 1.34(t, CH₃), 1.70-1.86(m, 1H), 1.97-2.10(m, 1H), 2.10-2.23(m, 1H), 2.48-2.62(m, 1H), 4.24(k, CH₂-CH₂), 4.30-4.50(m,3H, H4'+H5'), 5.22(s, CH₂-0). ¹³c NMR(CDCl₃, 75 MHz)δ : 14.23, 24.81, 33.20, 64.50, 67.36, 67.87, 79.55, 88.06, 94.13, 134.95, 144.05, 152.21, 154,94, 162.09.

### Example 17

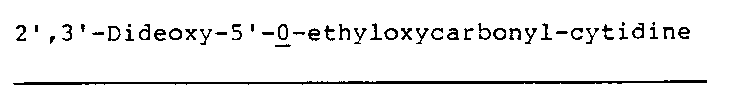

N⁴-Benzyloxycarbonyl-5'-0-ethyloxycarbonyl-2',3'-dideoxy-cytidine (0.0350 g, 8.387x10⁻⁵ mole) was added to a suspension of palladium on charcoal (5% Pd, 0.0040 g) in ethanol (1.0 ml). The air was replaced with nitrogen by repeated suction and addition of nitrogen. Hydrogen gas was added to the evacuated flask (15 ml flask) with a gastight syringe (5 ml). The reaction flask was shaken with this hydrogen pressure (1/3 atm) for 1 hour. Thin layer chromatography revealed partial consumption of the substrate and formation of a more polar product. The reaction slowed down after a while and the hydrogen pressure was increased to 1 atm. After a further 30 minutes more palladium on charcoal was added (0.0200 g) and the reduction continued until almost all the substrate was consumed (TLC) (2 hours).
The solvent was evaporated and the resulting black (charcoal) solid was subjected to a combined filtration and chromatography on a silica column. The eluents were chloroform, chloroform:ethanol 99:1 and chloroform:ethanol 9:1.
Yield: 0.0080 g (38.9 %) glassy material. ¹HNMR(CDCl₃ 300 MHz)δ : 1.33(t, CH₃), 1.65-1.85(m, 1H), 1.90-2.18(m, 1H), 2.40-2.55(m, 1H), 4.23(k, CH₂-CH₃), 4.28-4.43(m, 3H, H4'+H5'), 5.74(d, H5, J 7.44 Hz), 6.07(dd, 1H, H1'), 7.78(d, 1H, H6, J 7.44 Hz), 5.2-7.3(very broad, 2H, NH₂). ¹³C NMR(CDCl₃, 75 MHz, pulse delay 3s)δ : 14.24, 25.31, 32.99, 64.39, 67.90, 78.68, 87.36, 93.54, 140.90, 154.99, 155.87, 165.63.

### Example 18

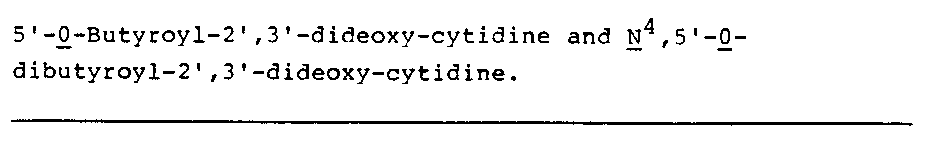

2',3'-Dideoxy-cytidine (0.0200 g, 9.467x10⁻⁵ mole) and N,N-dimethylaminopyridine (0.0116 g, 9.467x10⁻⁵ mole) were dissolved in a mixture of pyridine (1 ml) and dichloromethane (1 ml). The resulting mixture was cooled to 0°C and n-butyric anhydride (0.0236 g, 1.420x10⁻⁴ mole) (95%) was added with a syringe. The mixture was stirred at room temperature for 16 hours, water (2 ml) was added. Water and organic solvents were removed by high vacuum evaporation. The products were purified by chromatography on a silica column with chloroform:ethanol 9:1 as eluent.
5'-0-butyroyl-2',3'-dideoxy-cytidine
Yield: 0.0168 g (47.0 %). ¹HNMR(CDCl₃, 100 MHz) δ : 0.96(t, CH₃), 1.47-1.83(m, 1H), 1.68(k, CH₂), 1.83-2.20(m, 2H), 2.20-2.67(m, 1H), 2.35(t, CH₂), 4.35(broad, 3H, H4'+H5'), 5.76(d, 1H, H5, J 7.3 Hz), 6.04(dd, 1H, H1'), 5.5-7.2(very broad, 2H, NH₂), 7.73(d, 1H, H6).
N⁴,5'-0-dibutyroyl-2',3'-dideoxy-cytidine
Yield: 0.0021 g (4.1 %). Oil. ¹HNMR(CDCl₃ 100 MHz)δ : 0.98(t, CH₃), 1.00(t, CH₃), 1.7(2xk, 2-CH₂), 2.0-2.5(2xt, 2-CH₂), 4.37 (broad, 3H, H4'+H5'), 6.05(dd, 1H, H1'), 7.42(d, 1H, H5, J 7.8 Hz), 8.18(d, 1H, H6, J 7.8 Hz), 8.0(broad, 1H, NH), H2' and H3' obscured by other peaks,

### Example 19

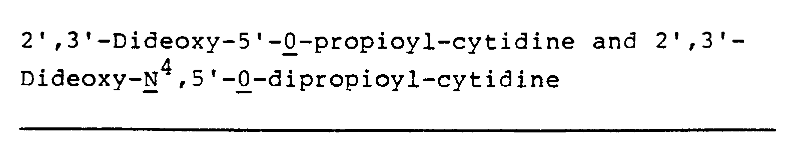

2',3'-Dideoxy-cytidine (0.0200 g, 9.467x10⁻⁵ mole) and N,N-dimethylaminopyridine (0.0116 g, 9.467x10⁻⁵ mole) were dissolved in a mixture of pyridine (1 ml) and dichloromethane (1 ml). The resulting mixture was cooled to 0°C and propionic anhydride (0.0185 g, 1.42x10⁻⁴ mole) was added with a syringe. The mixture was stirred at room temperature for 14 hours, water (2 ml) was added. Water and organic solvents were removed by high vacuum evaporation. The products were purified by chromatography on a silica column with chloroform:ethanol 9:1 as eluent.
2',3'-Dideoxy-N⁴-5'-0-dipropioyl-cytidine
Yield: 0.0132 g (43.1 %). Oil. ¹HNMR(CDCl₃, 100MHz)δ : 1.19(t, 2CH₃), 1.43-2.78 (several multiplets, 4H, H2'+H3'), 2.46(2xk, 2CH₂), 4.38 (broad, 3H, H4'+H5'), 6.60(dd, 1H, H1'), 7.44(d, 1H, H5, J 7.3 Hz), 6.19(d, 1H, H6, J 7.3 Hz), 9.0 (broad, 1H, NH).
2',3'-Dideoxy-5'-0-propioyl-cytidine
Yield: 0.0085 g (33.5 %). Oil. ¹HNMR(CDCl₃, 100 MHz).δ : 1.18(t, CH₃), 1.43-2.70 (several multiplets 4H, H2'+H3'), 2.40(k, CH₂), 4.33(broad, 3H, H4'+H5'), 5.73(d, 1H, H5, J 7.8 Hz), 6.50(dd, 1H, H1'), 7.79(d, 1H, H6, J 7.8 Hz), 5.0-7.3(very broad, 2H, NH₂).

### Pharmaceutical Example A

### Preparation of capsules for oral use

| | |
|---|---|
| 5'-0-Butyryl-2',3'-dideoxy-adenosine | 50 mg |
| Amylum maydis | q.s. |

The powder is mixed and filled into hard gelatin capsules (Capsugel Size 00).

### Pharamceutical Example B

### Preparation of an ointment

| | |
|---|---|
| N⁶,5'-0-Dibenzoyl-2',3'-dideoxy-adenosine | 1 g |
| Liquid paraffin | 100 g |
| White soft paraffin | to 1000 g |

White soft paraffin was melted and incorporated into the liquid paraffin and stirred until the mixture was cold. N⁶,5'-0-di-benzoyl-2',3'-dideoxy-adenosine was triturated with a portion of the basis and gradually the remainder of the basis was incorporated. The ointment was filled into lacquered aluminium tubes (20 g) and sealed. The ointment contained 0.1 % N⁶,5'-0-dibenzoyl-2',3'-dideoxy-adenosine.

### Pharmaceutical Example C

### Suspension for parenteral administration

| | |
|---|---|
| 2',3'-Dideoxy-5'-0-palmitoyl-cytidine | 200 gram |
| Polysorbate 80 | 3 gram |
| Sorbitol | 400 gram |
| Benzyl alcohol | 8 gram |
| Water | ad 1000 ml |
| 1M HCl | q.s. |

Polysorbate 80, Sorbitol and benzyl alcohol were dissolved in 500 ml distilled water. 2',3'-Dideoxy-5'-0-palmitoyl-cytidine was screened through a 0.15 mm sieve and dispersed in the solution under vigorous stirring. The pH was adjusted to 4.5 by dropwise addition of 1M HCl. Water was added to 1000 ml, the suspension was filled in 1 ml vials The vials were sterilized by -radiation. Each vial contained 200 mg 2',3'-dideoxy-5-0-palmitoyl-cytidine.

### Pharmaceutical Example D

### Preparation of tablets

| | Gram |
|---|---|
| N⁴,5'-0-diacetyl-2',3'-dideoxy-cytidine | 200 |
| Lactose | 85 |
| Polyvinylpyrrolidone | 5 |
| Starch | 42 |
| Talcum powder | 15 |
| Magnesium stearate | 3 |

N⁴,5'-0-Diacetyl-2',3'-dideoxy-cytidine and lactose were screened through a 0.15 mm sieve and mixed together for 10 minutes. The mixed powder was wetted with an aqueous solution of polyvinyl-pyrrolidone. The mass was granulated, and the dried (40 °C) granulate was mixed with starch, talcum powder and magnesium stearate. The granulate was compressed into tablets. The tablet diameter was 11 mm, the tablet was 350 mg and each tablet contained 200 mg N⁴,5'-0-diacetyl-2',3'-dideoxy-cytidine.

### Pharmaceutical Example E

### Preparation of a suspension for rectal administration

Methyl parahydroxybenzoate (70 mg) and propyl parahydroxybenzoate (15 mg) were dissolved in water (100 ml) at 90 °C. After cooling to 30 °C methyl cellulose (2g) was added and the mixture was agitated for 3 hours. 1 gram N⁴-benzoyl-2',3'-dideoxy-cytidine was screened through a 0.15 mm sieve, and dispersed in the solution under vigorous stirring. The suspension was filled in a 100 ml tube. The suspension contained 10 mg N⁴-benzoyl-2',3'-dideoxy-cytidine/ml.

### Pharmaceutical Example F

### Preparation of oral suspension

| | Gram |
|---|---|
| 2',3'-dideoxy-N⁴-hexanoyl-cytidine | 10 |
| Carboxymethyl cellulose | 1.5 |
| Sorbitol | 200 |
| Sodium benzoate | 1.0 |
| Orange essence | 0.3 |
| Apricot essence | 0.7 |
| Ethanol | 50 |
| Water | 236.5 |

Carboxymethyl cellulose, sorbitol and sodium benzoate were dissolved in water with stirring for 2 hours. A solution of the essences in ethanol was added. 2',3'-Dideoxy-N⁴-hexanoyl-cytidine was screened through a 0.15 mm sieve and dispersed in the solution under vigorous stirring. The suspension (10 gram) was filled in a 20 ml tube. Each tube contained 200 mg 2',3'-dideoxy-N⁴-hexanoyl-cytidine.

### Pharmaceutical Example G

### Preparation of injection solution

10 mg 5'-0-acetyl-2',3'-dideoxy-cytidine were dissolved in 10 ml 0.9 % sodium chloride. pH was adjusted to 4.5 with 1N HCl. The solution was sterile filtered and filled into a 10 ml vial. The solution contained 1 mg 5'-0-acetyl-2',3'-dideoxy-cytidime/ml.

### Pharmaceutical Example H

### Preparation of tablets (controlled release formulation)

| | Gram |
|---|---|
| 2',3'-Dideoxy-5'-0-ethyloxycarbonyl-cytidine | 500 |
| Hydroxypropylmethylcellulose (Methocel K15) | 120 |
| Lactose | 45 |
| Povidone | 30 |
| Magnesium stearate | 5 |

2',3'-Dideoxy-5'-0-ethyloxycarbonyl-cytidine, hydroxypropylmethylcellulose and lactose were mixed together for 20 minutes and granulated with a solution of povidone. Magnesium stearate was added and the mixture was compressed into tablets. The tablet diameter was 13 mm, the tablet weight was 700 mg and each tablet contained 500 mg 2',3'-dideoxy-5'-0-ethyloxycarbonyl-cytidine.

## Claims

1. Use of compounds of formula (I) 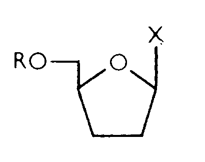 wherein R is a hydrogen atom or a physiologically acceptable acyl group of formula R¹.CO- or R¹.O.CO-, R¹ being an optionally substituted alkyl or aryl group, and X is selected from 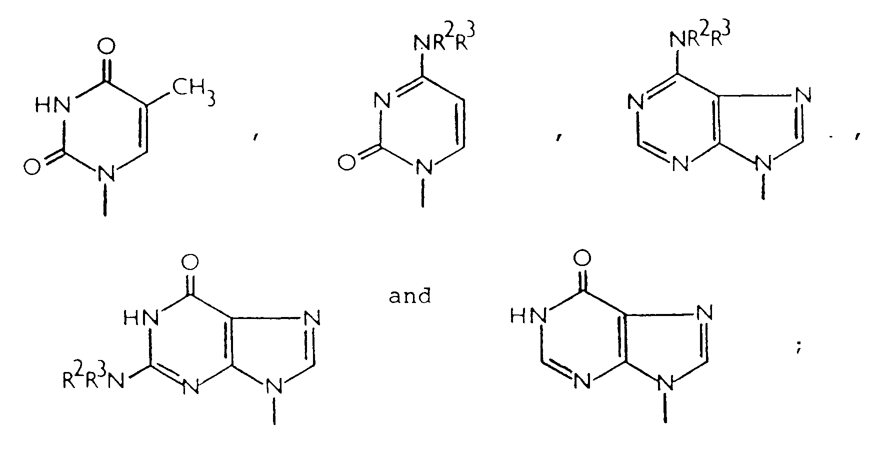 wherein R² and R³, which may be the same or different, are each a hydrogen atom or a physiologically acceptable acyl group of formula R⁴.CO- or R⁴.O.CO-, R⁴ being an optionally substituted alkyl or aryl group, with the proviso that at least one of R and R² must be an acyl group, and/or salts thereof in the preparation of a pharmaceutical composition for combatting neurological disorders caused by neurotropic viruses.

2. Use as claimed in claim 1 wherein R² and R³ in the compound of formula (I) are hydrogen atoms and R is a group R¹.O.CO-, R¹ being an optionally substituted alkyl or aryl group.

3. Use as claimed in claim 1 wherein R² in the compound of formula (I) is a group of formula R⁴.CO or R⁴.O.CO-, R⁴ being an optionally substituted alkyl or aryl group, R³ is a hydrogen atom or a group as defined for R² and R is a hydrogen atom or a group of formula R¹.CO or R¹.O.CO-, R¹ being an optionally substituted alkyl or aryl group.

4. Use as claimed in any preceding claim wherein R, R² and R³ in the compound of formula (I) are independently selected from hydrogen atoms and C₁₋₂₀ acyl groups.

5. Use as claimed in any preceding claim wherein X in the compound of formula (I) is a thymine radical.

6. Use as claimed in any preceding claim wherein the pharmaceutical composition further comprises an antiviral agent selected from acyclovir, phosphonoformate, suramin, Evans Blue, interferons and 3'-azido-3'- deoxythymidine.

7. An orally administrable pharmaceutical composition comprising as active ingredient one or more compounds of formula (I) 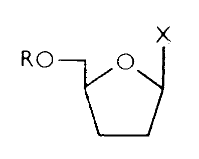 wherein R is a hydrogen atom or a physiologically acceptable acyl group of formula R¹.CO- or R¹.O.CO-, R¹ being an optionally substituted alkyl or aryl group, and X is selected from 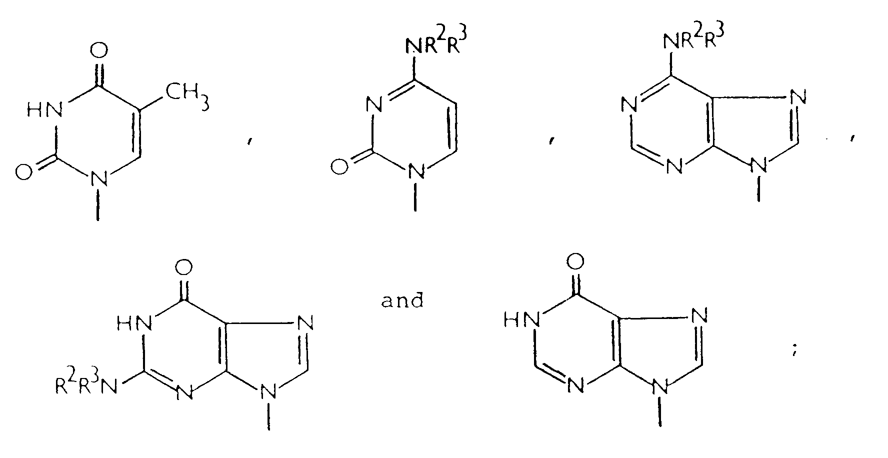 wherein R² and R³, which may be the same or different, are each a hydrogen atom or a physiologically acceptable acyl group of formula R⁴.CO- or R⁴.O.CO-, R⁴ being an optionally substituted alkyl or aryl group, with the proviso that at least one of R and R² must be an acyl group and when R is a hydrogen atom and X is a cytosine moiety in which R² is a group R⁴.CO, then R³ is other than a hydrogen atom, and/or salts thereof.

8. A composition as claimed in claim 7 wherein R² and R³ in the compound of formula (I) are hydrogen atoms and R is a group R¹.O.CO-, R¹ being an optionally substituted alkyl or aryl group.

9. A composition as claimed in claim 7 wherein R² in the compound of formula (I) is a group of formula R⁴.CO or R⁴.O.CO-, R⁴ being an optionally substituted alkyl or aryl group, R³ is a hydrogen atom or a group as defined for R² and R is a hydrogen atom or a group of formula R¹.CO or R¹.O.CO-, R¹ being an optionally substituted alkyl or aryl group.

10. A composition as claimed in any of claims 7 to 9 wherein R, R² and R³ in the compound of formula (I) are independently selected from hydrogen atoms and C₁₋₂₀ acyl groups.

11. A composition as claimed in any of claims 7 to 10 wherein X in the compound of formula (I) is a thymine radical.

12. A composition as claimed in any claims 7 to 11 which further comprises an antiviral agent selected from acyclovir, phosphonoformate, suramin, Evans Blue, interferons and 3'-azido-3'- deoxythymidine.

13. Compounds of formula (I) 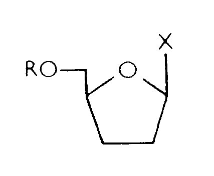 wherein R is a hydrogen atom or a physiologically acceptable acyl group of formula R¹.CO- or R¹.O.CO-, R¹ being an optionally substituted alkyl or aryl group, and X is 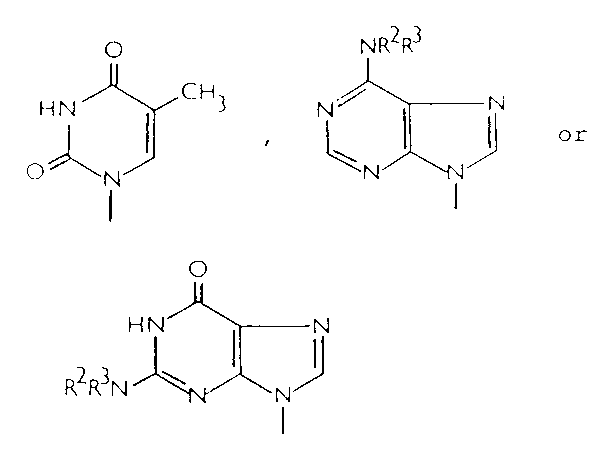 wherein R² and R³, which may be the same or different, are each a hydrogen atom or a physiologically acceptable acyl group of formula R⁴.CO- or R⁴.O.CO-, R⁴ being an optionally substituted alkyl or aryl group, with the proviso that at least one of R and R² must be an acyl group, with the further proviso that when R is an acetyl group then X is not a thymine radical; when R is a benzoyl group then X is not a thymine radical and when R is a 3-(trifluoromethyl)-benzoyl group then X is not an N-unsubstituted adenine radical; and salts thereof.

14. Compounds as claimed in claim 13 wherein R² and R³ in the group X are hydrogen atoms and R is a group R¹.O.CO-, R¹ being an optionally substituted alkyl or aryl group.

15. Compounds as claimed in claim 13 wherein R² in the group X is a group of formula R⁴.CO- or R⁴.O.CO-, R⁴ being an optionally substituted alkyl or aryl group, R³ in the group X is a hydrogen atom or a group as defined for R² and R is a hydrogen atom or a group of formula R¹.CO- or R¹.O.CO-, R¹ being an optionally substituted alkyl or aryl group.

16. A process for the preparation of a compound of formula (I) as defined in claim 13 or a salt thereof which comprises reaction of a compound of formula (II) 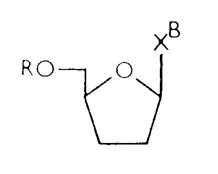 [wherein R is as defined in claim 13 and X^{B} is as defined in claim 13 for X except that R and R² and/or R³ in the group X may each additionally represent a protecting group, with the proviso that at least one of R, R² and R³ is a hydrogen atom] with an acylating agent serving to introduce an acyl group R¹CO-, R¹OCO-, R⁴CO- or R⁴OCO-, R¹ and R⁴ each being an optionally substituted alkyl or aryl group, followed where required by removal of any protecting groups and/or unwanted acyl substituents.

## Patentansprüche

1. Verwendung der Verbindungen der Formel (I) 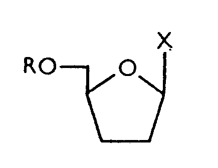 worin R für ein Wasserstoffatom oder eine physiologisch verträgliche Acylgruppe der Formel R¹.CO- oder R¹.O.CO-steht, wobei R¹ eine gegebenenfalls substituierte Alkyl- oder Arylgruppe ist und X unter 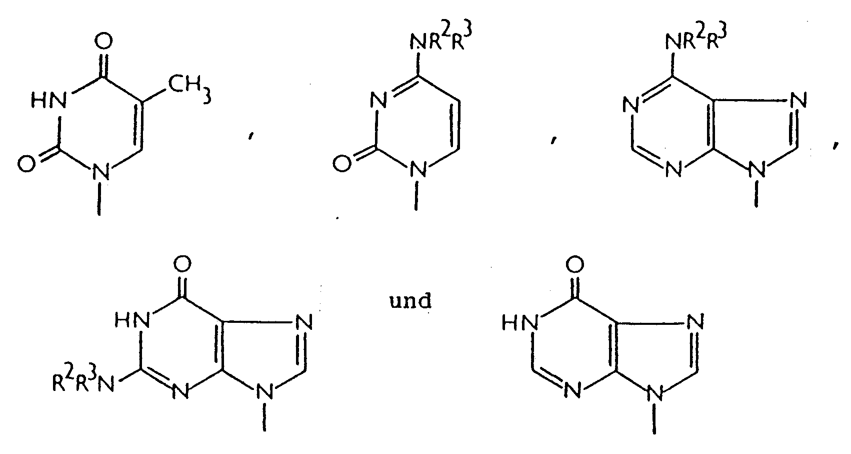 ausgewählt ist, worin R² und R³, die gleich oder verschieden sein können, jeweils ein Wasserstoffatom oder eine physiologisch verträgliche Acylgruppe der Formel R⁴.CO- oder R⁴.O.CO- bedeuten, wobei R⁴ eine gegebenenfalls substituierte Alkyl- oder Arylgruppe ist, mit der Maßgabe, daß zumindest eines von R und R² eine Acylgruppe sein muß, und/oder von deren Salzen bei der Herstellung einer pharmazeutischen Zusammensetzung für die Bekämpfung von neurologischen Störungen, die durch neurotrope Viren verursacht werden.

2. Verwendung gemäß Anspruch 1, worin R² und R³ in der Verbindung der Formel (I) Wasserstoffatome sind und R für eine Gruppe R¹.O.CO- steht, wobei R¹ eine gegebenenfalls substiuierte Alkyl- oder Arylgruppe ist.

3. Verwendung gemäß Anspruch 1, worin R² in der Verbindung der Formel (I) eine Gruppe der Formel R⁴.CO oder R⁴.O.CO-ist, wobei R⁴ eine gegebenenfalls substuierte Alkyl- oder Arylgruppe ist, R³ für ein Wasserstoffatom oder eine Gruppe wie für R² definiert steht und R ein Wasserstoffatom oder eine Gruppe der Formel R¹.CO oder R¹.O.CO- darstellt, wobei R¹ eine gegebenenfalls substituierte Alkyl oder Arylgruppe ist.

4. Verwendung gemäß einem der vorhergehenden Ansprüche, worin R, R² und R³ in der Verbindung der Formel (I) unabhängig unter Wasserstoffatomen und C₁₋₂₀-Acylgruppen ausgewählt sind.

5. Verwendung gemäß einem der vorhergehenden Ansprüche, worin X in der Verbindung der Formel (I) ein Thyminrest ist.

6. Verwendung gemäß einem der vorhergehenden Ansprüche, worin die pharmazeutische Zusammensetzung außerdem ein antivirales Mittel, ausgewählt unter Acyclovir, Phosphonoformiat, Suramin, Evans Blue, Interferonen und 3'-Azido-3'-desoxythymidin, enthält.

7. Oral verabreichbare pharmazeutische Zusammensetzung, enthaltend als Wirkstoff eine oder mehrere Verbindungen der Formel (I) 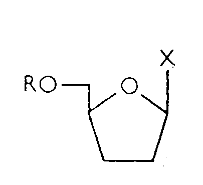 worin R ein Wasserstoffatom oder eine physiologisch verträgliche Acylgruppe der Formel R¹.CO- oder R¹.O.CO-darstellt, wobei R¹ eine gegebenenfalls substituierte Alkyl- oder Arylgruppe ist und X unter 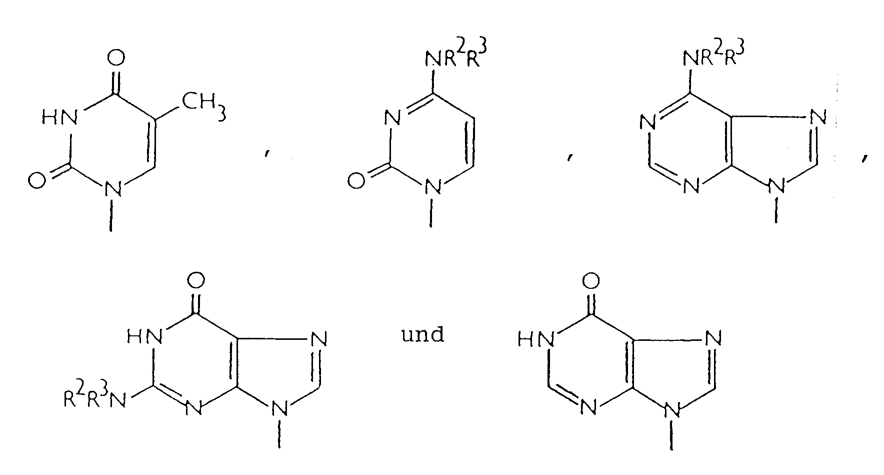 ausgewählt ist, worin R² und R³, die gleich oder verschieden sein können, jeweils ein Wasserstoffatom oder eine physiologisch verträgliche Acylgruppe der Formel R⁴.CO- oder R⁴.O.CO- bedeuten, wobei R⁴ eine gegebenenfalls substituierte Alkyl- oder Arylgruppe ist, mit der Maßgabe, daß zumidenst eines von R und R² eine Acylgruppe sein muß, und wenn R ein Wasserstoffatom ist und X ein Cytosinteil bedeutet, worin R² für eine Gruppe der Formel R⁴.CO steht, R³ von einem Wasserstoffatom verschieden ist, und/oder deren Salze.

8. Zusammensetzung gemäß Anspruch 7, worin R² und R³ in der Verbindung der Formel (I) Wasserstoffatome bedeuten und R für eine Gruppe R¹.O.CO- steht, wobei R¹ eine gegebenenfalls substituierte Alkyl- oder Arylgruppe ist.

9. Zusammensetzung gemäß Anspruch 7, worin R² in der Verbindung der Formel (I) für eine Gruppe der Formel R⁴.CO oder R⁴.O.CO- steht, wobei R⁴ eine gegebenenfalls substituierte Alkyl- oder Arylgruppe ist, R³ ein Wasserstoffatom oder eine Gruppe wie für R² definiert bedeutet und R ein Wasserstoffatom oder eine Gruppe der Formel R¹CO oder R¹.O.CO- darstellt, wobei R¹ eine gegebenenfalls substituierte Alkyl- oder Arylgruppe ist.

10. Zusammensetzung gemäß einem der Ansprüche 7 bis 9, worin R, R² und R³ in der Verbindung der Formel (I) unabhängig unter Wasserstoffatomen und C₁₋₂₀-Acylgruppen ausgewählt sind.

11. Zusammensetzung gemäß einem der Ansprüche 7 bis 10, worin X in der Verbindung der Formel (I) ein Thyminrest ist.

12. Zusammensetzung gemäß einem der Ansprüche 7 bis 11, die weiterhin ein antivirales Mittel, ausgewählt unter Acyclovir, Phosphonoformiat, Suramin, Evans Blue, Interferonen und 3'-Azido-3'-desoxythymidin, enthält.

13. Verbindungen der Formel (I) 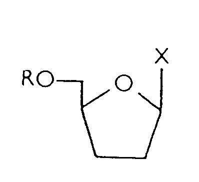 worin R ein Wasserstoffatom oder eine physiologisch verträgliche Acylgruppe der Formel R¹.CO- oder R¹.O.CO-bedeutet, wobei R¹ eine gegebenenfalls substituierte Alkyl- oder Arylgruppe ist und X für 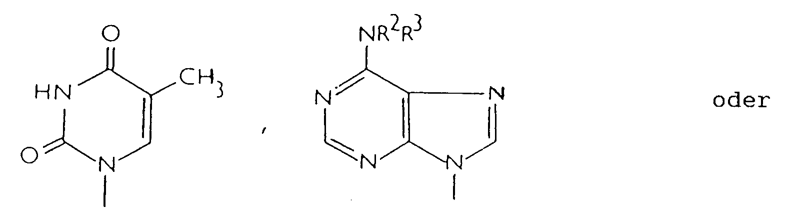 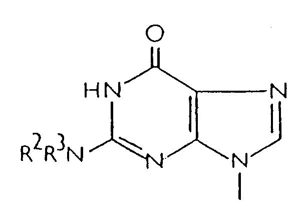 stellt, worin R² und R³, die gleich oder verschieden sein können, jeweils ein Wasserstoffatom oder eine physiologisch verträgliche Acylgruppe der Formel R⁴.CO- oder R⁴.O.CO- bedeuten, wobei R⁴ eine gegebenenfalls substituierte Alkyl- oder Arylgruppe ist, mit der Maßgabe, daß zumindest eines von R und R² eine Ayclgruppe sein muß, mit der weiteren Maßgabe, daß, wenn R für eine Acetylgruppe steht, X keinen Thyminrest bedeutet; wenn R eine Benzoylgruppe ist, X keinen Thyminrest bedeutet und wenn R eine 3-(Trifluormethyl)-benzoylgruppe ist, X kein N-unsubstituierter Adeninrest ist; und deren Salze.

14. Verbindungen gemäß Anspruch 13, worin R² und R³ in der Gruppe X Wasserstoffatome sind und R eine Gruppe R¹.O.CO-bedeutet, wobei R¹ eine gegebenenfalls substituierte Alkyl- oder Arylgruppe ist.

15. Verbindungen gemäß Anspruch 13, worin R² in der Gruppe X eine Gruppe der Formel R⁴.CO- oder R⁴.O.CO- ist, wobei R⁴ eine gegebenenfalls substituierte Alkyl- oder Arylgruppe bedeutet, R³ in der Gruppe X ein Wasserstoffatom oder eine Gruppe wie für R² definiert darstellt und R ein Wasserstoffatom oder eine Gruppe der Formel R¹.CO- oder R¹.O.CO- ist, wobei R¹ eine gegebenenfalls substituierte Alkyl- oder Arylgruppe darstellt.

16. Verfahren zur Herstellung einer Verbindung der Formel (I), wie in Anspruch 13 definiert, oder eines Salzes hiervon, das die Umsetzung einer Verbindung der Formel (II) 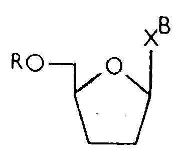 [worin R wie in Anspruch 13 definiert ist und X^{B} wie in Anspruch 13 für X definiert ist, mit der Ausnahme, daß R und R² und/oder R³ in der Gruppe X zusätzlich eine Schutzgruppe bedeuten können, mit der Maßgabe, daß zumindest eines von R, R² und R³ ein Wasserstoffatom ist] mit einem Acylierungsmittel, das der Einführung einer Acylgruppe R¹CO-, R¹OCO-, R⁴CO- oder R⁴OCO- dient, wobei R¹ und R⁴ jeweils eine gegebenenfalls substituierte Alkyl- oder Arylgruppe bedeuten, und erforderlichfalls die anschließende Entfernung irgendwelcher Schutzgruppen und/oder unerwünschter Acylsubstituenten umfaßt.

## Revendications

1. Utilisation de composés de formule (I) : 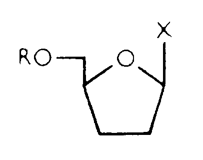 dans laquelle R représente un atome d'hydrogène ou un groupe acyle physiologiquement acceptable de formule R¹.CO- ou R¹.O.CO-, R¹ représentant un groupe alkyle ou aryle éventuellement substitué, et X est choisi. parmi : 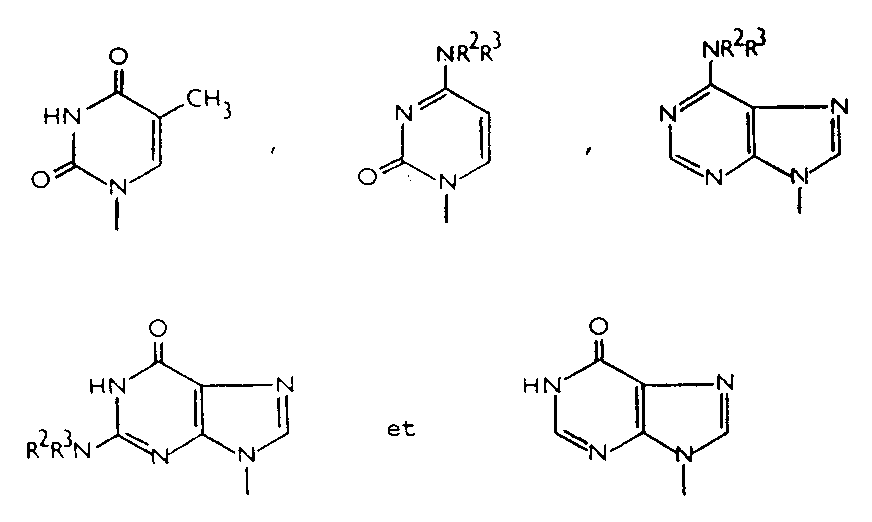 dans lesquels R² et R³ qui peuvent être identiques ou différents, représentent chacun un atome d'hydrogène ou un groupe acyle physiologiquement acceptable de formule R⁴.CO- ou R⁴.O.CO-, R⁴ représentant un groupe alkyle ou aryle éventuellement substitué, à condition qu'au moins l'un des groupes R et R² représente un groupe acyle, et/ou les sels de ceux-ci, pour la préparation d'une composition pharmaceutique pour combattre des troubles neurologiques dus à des virus neurotropes.

2. Utilisation selon la revendication 1, dans laquelle R² et R³ dans le composé de formule (I), représentent des atomes d'hydrogène, et R représente un groupe R¹.O.CO-, R¹ représentant un groupe alkyle ou aryle éventuellement substitué.

3. Utilisation selon la revendication 1, dans laquelle R² dans le composé de formule (I), représente un groupe de formule R⁴.CO ou R⁴.O.CO-, R⁴ représentant un groupe alkyle ou aryle éventuellement substitué, R³ représente un atome d'hydrogène ou un groupe tel que défini pour R², et R représente un atome d'hydrogène ou un groupe de formule R¹.CO ou R¹.O.CO-, R¹ représentant un groupe alkyle ou aryle éventuellement substitué.

4. Utilisation selon l'une quelconque des revendications précédentes, dans laquelle R, R² et R³ dans le composé de formule (I), sont indépendamment choisis parmi un atome d'hydrogène et les groupes acyle en C₁-C₂₀.

5. Utilisation selon l'une quelconque des revendications précédentes, dans laquelle X dans le composé de formule (I), représente un radical de thymine.

6. Utilisation selon l'une quelconque des revendications précédentes, dans laquelle la composition pharmaceutique comprend en outre un agent antiviral choisi parmi l'acyclovir, un phosphonoformate, la suramine, l'Evans Blue, les interférons et la 3'-azido-3'-désoxythymidine.

7. Composition pharmaceutique administrable par voie orale, comprenant, comme ingrédient actif, un ou plusieurs composés de formule (I) : 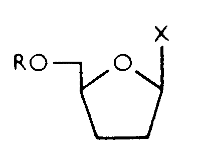 dans laquelle R représente un atome d'hydrogène ou un groupe acyle physiologiquement acceptable de formule R¹.CO- ou R¹.O.CO-, R¹ représentant un groupe alkyle ou aryle éventuellement substitué, et X est choisi parmi : 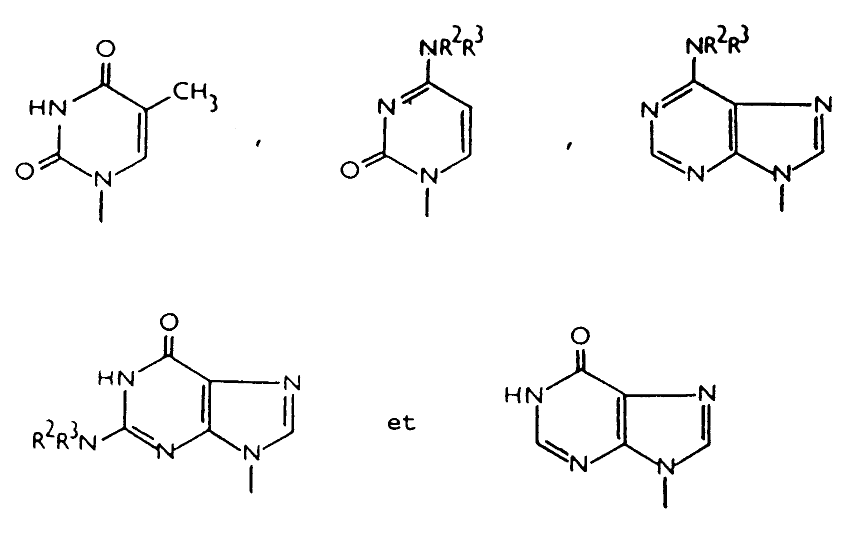 dans lesquels R² et R³ qui peuvent être identiques ou différents, représentent chacun un atome d'hydrogène ou un groupe acyle physiologiquement acceptable de formule R⁴.CO- ou R⁴.O.CO-, R⁴ représentant un groupe alkyle ou aryle éventuellement substitué, à condition qu'au moins l'un des groupes R et R² représente un groupe acyle, et lorsque R représente un atome d'hydrogène et X représente un groupe dérivé de cytosine dans lequel R² représente un groupe R⁴.CO, R³ est alors autre qu'un atome d'hydrogène, et/ou les sels de ceux-ci.

8. Composition selon la revendication 7, dans laquelle R² et R³ dans le composé de formule (I), représentent des atomes d'hydrogène, et R représente un groupe R¹.O.CO-, R¹ représentant un groupe alkyle ou aryle éventuellement substitué.

9. Composition selon la revendication 7, dans laquelle R² dans le composé de formule (I) représente un groupe de formule R⁴.CO ou R⁴.O.CO-, R⁴ représentant un groupe alkyle ou aryle éventuellement substitué, R³ représente un atome d'hydrogène ou un groupe tel que défini pour R², et R représente un atome d'hydrogène ou un groupe de formule R¹.CO ou R¹.O.CO-, R¹ représentant un groupe alkyle ou aryle éventuellement substitué.

10. Composition selon l'une quelconque des revendications 7 à 9, dans laquelle R, R² et R³ dans le composé de formule (I), sont indépendamment choisis parmi un atome d'hydrogène et les groupes acyle en C₁-C₂₀.

11. Composition selon l'une quelconque des revendications 7 à 10, dans laquelle X dans le composé de formule (I), représente un radical de thymine.

12. Composition selon l'une quelconque des revendications 7 à 11, comprenant en outre un agent antiviral choisi parmi l'acyclovir, un phosphonoformiate, la suramine, l'Evans Blue, les interférons et la 3'-azido-3'-désoxythymidine.

13. Composés de formule (I) : 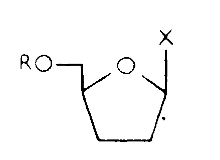 dans laquelle R représente un atome d'hydrogène ou un groupe acyle physiologiquement acceptable de formule R¹.CO- ou R¹.O.CO-, R¹ représentant un groupe alkyle ou aryle éventuellement substitué, et X représente : 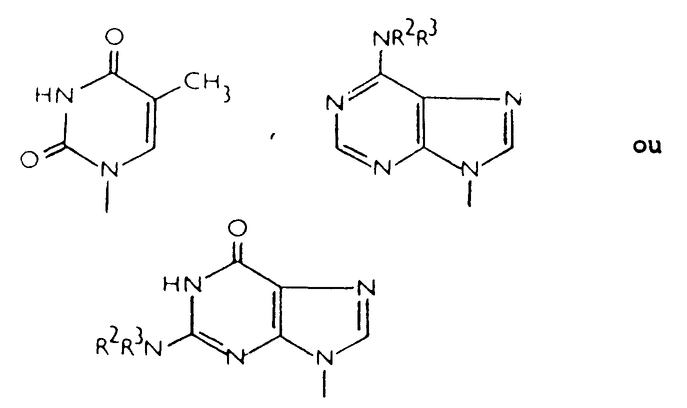 dans lesquels R² et R³ qui peuvent être identiques ou différents, représentent chacun un atome d'hydrogène ou un groupe acyle physiologiquement acceptable de formule R⁴.CO- ou R⁴.O.CO-, R⁴ représentant un groupe alkyle ou aryle éventuellement substitué, à condition qu'au moins l'un des groupes R et R² représente un groupe acyle, et en outre à condition que lorsque R représente un groupe acétyle, X ne représente alors pas un radical de thymine ; lorsque R représente un groupe benzoyle, X ne représente alors pas un radical de thymine, et lorsque R représente un groupe 3-(trifluorométhyl)-benzoyle, X ne représente alors pas un radical d'adénine N-non substituée ; et les sels de ceux-ci.

14. Composés selon la revendication 13, dans lesquels R² et R³ dans le groupe X, représentent des atomes d'hydrogène, et R représente un groupe R¹.O.CO-, R¹ représentant un groupe alkyle ou aryle éventuellement substitué.

15. Composés selon la revendication 13, dans lesquels R² dans le groupe X représente un groupe de formule R⁴.CO- ou R⁴.O.CO-, R⁴ représentant un groupe alkyle ou aryle éventuellement substitué, R³ dans le groupe X représentant un atome d'hydrogène ou un groupe tel que défini pour R², et R représente un atome d'hydrogène ou un groupe de formule R¹.CO- ou R¹.O.CO-, R¹ représentant un groupe alkyle ou aryle éventuellement substitué.

16. Procédé de préparation d'un composé de formule (I) selon la revendication 13, ou d'un sel de celui-ci, selon lequel on fait réagir un composé de formule (II) : 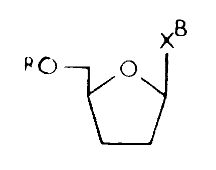 (dans laquelle R est tel que défini dans la revendication 13, et X^{B} est tel que défini dans la revendication 13 pour X, à l'exception que R et R² et/ou R³ dans le groupe X, peuvent en outre représenter chacun un groupe protecteur, à condition qu'au moins l'un des groupes R, R² et R³ représente un atome d'hydrogène) avec un agent acylant servant à introduire un groupe acyle R¹CO-, R¹OCO-, R⁴CO- ou R⁴OCO-, R¹ et R⁴ représentant chacun un groupe alkyle ou aryle éventuellement substitué, puis, lorsque cela est nécessaire, on élimine les groupes protecteurs et/ou les substituants acyle non souhaités.
